# EUROPEAN PATENT APPLICATION

(11) **EP 4 822 354 A1**
(43) Date of publication of application: **14.10.2026**
(21) Application number: 25170012.6
(22) Date of filing: 11.04.2025
(51) Int. Cl.: G01N 33/68, G16B 40/10

(54) **SYSTEM AND METHOD FOR PATHOGEN IDENTIFICATION FROM MALDI SPECTRA**

(71) Applicant: Selucky, Pavel, 60300 Brno (CZ); Ruzicka, Viktor, 793 44 Horni mesto (CZ); Luks, David, 66401 Bilovice nad Svitavou (CZ); Svub, Miroslav, 66441 Troubsko (CZ)
(72) Inventor: Selucky, Pavel, 60300 Brno (CZ); Ruzicka, Viktor, 793 44 Horni mesto (CZ); Luks, David, 66401 Bilovice nad Svitavou (CZ); Svub, Miroslav, 66441 Troubsko (CZ)
(74) Representative: Harber IP s.r.o.

(57) **Abstract**

A novel system and method are disclosed for identifying microbial pathogens from MALDI-TOF mass spectrometry data using a Fourier Neural Operator (FNO) approach deployed on a cloud-based platform. The invention treats each MALDI spectrum as a global function, performing baseline correction, smoothing, and binning. A neural operator architecture applies learned transformations in the frequency domain, capturing wide-range spectral dependencies. The final global feature vector is classified into pathogen species. This yields more robust performance than local convolutional filters, especially for subtle distinctions among closely related microorganisms. The cloud-based digital platform supports high-throughput sample uploads and real-time inference, enabling a scalable, centralized service that streamlines clinical or research workflows in microbiological diagnostics.

## Description

### TECHNICAL FIELD

The present invention relates to methods and systems for analyzing and classifying mass spectrometry data, based on the use of Fourier Neural Operators (FNO) to identify microbial pathogens from MALDI (Matrix-Assisted Laser Desorption/Ionization) spectra, preferably in a cloud-based environment.

### BACKGROUND ART

Matrix-Assisted Laser Desorption/Ionization Time-of-Flight (MALDI-TOF) mass spectrometry has become a standard technique in microbiological and clinical diagnostics. When a laser pulse strikes a matrix containing the microbial sample, molecular ions are produced and accelerated through a flight tube. The resulting mass spectrum-peaks of intensity versus m/z (mass-to-charge ratio)-serves as a signature of the organism, aiding identification at the species or subspecies level.

Traditional MALDI analyses often rely on matching spectral peaks to a reference library. However, expansions in microbial libraries and the emergence of novel organisms or strains highlight the need for more sophisticated, data-driven classification systems. Machine learning approaches, such as neural networks, hold promise for robust, automated identification, especially when dealing with large, heterogeneous datasets.

Conventional neural network models, such as Convolutional Neural Networks (CNNs), typically rely on local filters scanning across input data. While CNNs have proven efficient in many imaging tasks, MALDI spectra present specific challenges: peak shifts, baseline variations, wide dynamic range, and the importance of global spectral patterns. Overemphasis on local features can result in suboptimal classification, especially when distinguishing closely related microbes.

Existing MALDI classification solutions commonly mention local pattern matching or simpler neural architectures. Patent publications typically disclose either specialized hardware or library-based matching.

### SUMMARY OF THE INVENTION

One aspect of the present invention provides a (computer-implemented) method and system for identifying pathogens from MALDI spectra, comprising the step of processing the MALDI spectra by a Fourier Neural Operator (FNO) neural network.

Generally, the method of identifying a microbial pathogen according to the invention comprised the following steps:
- obtaining a MALDI mass spectrometry spectrum representing intensities at m/z;

- preprocessing said spectrum by at least one of baseline correcting, smoothing, and normalizing intensities;
- performing a lifting transform to map said one-dimensional intensities into a higher-dimensional feature representation (space);
- executing at least one Fourier layer that applies a forward Fourier transform to obtain transformed feature representation, multiplying the transformed feature representation by learned complex multipliers to obtain convoluted feature representation, and applying an inverse Fourier transform to the convoluted feature representation to obtain feature vectors;
- pooling feature vectors across bins to produce a global representation;
- classifying the global representation into a pathogen label.

In one embodiment, the method of the invention includes obtaining a MALDI mass spectrometry spectrum representing intensities at m/z and processing the MALDI spectrum as follows:
a) a preprocessing step including MALDI spectra wherein the preprocessing step comprises at least one of baseline correction, smoothing and normalization;
b) optionally a step of dividing the MALDI spectral data into intervals (bins) of m/z values and calculating an average intensity in each interval (bin);
c) processing of the pre-processed MALDI data by a Fourier Neural Operator Architecture which comprises several Fourier layers, wherein c1) a lifting layer maps the one-dimensional MALDI data into a higher-dimensional feature space and wherein the c2) Fourier layers transform the obtained features in the higher-dimensional feature space to a frequency domain,
d) processing the obtained features in the higher-dimensional feature space by a neural network applying learned complex multipliers,
e) processing the data obtained in step d) by inverse Fourier transformation to obtain feature vectors,
f) repeating the steps c2) to e) at least twice, preferably at least four times, wherein the input of the repeated step c2) are the data obtained in step e);
g) pooling feature vectors across bins to produce a global representation;
h) classifying the global representation into a pathogen label.

The Fourier Neural Operator processes a limited number of frequency modes to reduce computational complexity while preserving global coverage.

The method may include an adaptive iteration procedure in which the spectral convolution layers can be repeated multiple times for refinement.

The method of the invention can incorporate a data quality check to flag any spectra lacking distinct peaks or having an exceedingly low total intensity. Such checks reduce erroneous classifications. The user is then informed that the sample might need re-measurement or a more robust peak extraction approach. The FNO can also be extended to handle partial or truncated spectra, since the frequency domain transformations remain flexible so long as the input shape is consistent.

A significant advantage is that each frequency mode in the FNO provides "global coverage" of the spectral domain. In FNO terms, small amplitude changes in certain frequencies may shift intensities globally across bins, thereby enabling the model to detect subtle species-specific patterns that purely local kernel approaches can overlook.

Empirical results suggest that while a CNN might offer quick local pattern recognition, it can falter when minor shifts occur across widely spaced bins or when certain global patterns are needed to differentiate near-identical microbial species. FNO's spectral approach unifies these bins in a single global operation, showing improved consistency under such conditions.

The method of the invention is based on using a functional operator perspective, which enables a global coverage in the frequency domain captures patterns across the entire m/z range, as well as improved accuracy for closely related microbial species, better resilience to minor peak shifts, and robust performance even with partial spectral noise.

FNO-based models learn an operator mapping from a function space to a target space. By applying a global perspective in the frequency domain, FNOs capture relationships spanning the entire spectral range.

The FNO-based model is trained based on MALDI data and a corresponding assignment of a pathogen. The training data may include also additional parameters such as antibiotic sensitivity of the pathogen and virulence of the pathogen.

The trained model is then validated with validation data containing MALDI data and a corresponding assignment of a pathogen, optionally also the additional parameters.

The training process typically requires:
- A labeled dataset of MALDI spectra with known species.
- Splitting into training and validation sets.
- A suitable loss function, e.g. cross-entropy, possibly with class weights.
- Regularization methods such as dropout in spectral layers or weight decay in the optimizer.
- Possibly early stopping, if validation accuracy stagnates or declines.

The term "pathogen" or "microbial pathogen" generally refers to microorganisms, in particular to bacteria, fungi, protozoans, viroids and viruses.

In preferred embodiments, the method may be implemented with cloud deployment, enabling real-time pathogen detection across multiple remote sites. Furthermore, it enables excellent scalability - the model can handle large volumes of data on cloud servers.

By deploying the model on a cloud-based platform, laboratories can upload MALDI data for remote, high-throughput classification while benefiting from globally learned spectral dependencies.

Modern laboratories often desire a centralized digital platform for analyzing MALDI data. Deploying advanced AI models on a cloud-based system offers scalability, easy updates, and resource pooling for computationally intensive tasks. This approach can integrate with laboratory information systems (LIS) or hospital data networks seamlessly.

The cloud-based implementation simplifies updates. If new species or thousands more training examples become available, the FNO model can be retrained. Once validated, the updated model is deployed to the production environment. All participating labs seamlessly benefit from the improved model, eliminating the need for local software patches or GPU hardware upgrades.

A cloud-based implementation could have the following features and advantages: a digital platform where user labs can securely upload MALDI spectra; the FNO model runs inference, computing species probabilities for each sample; results are delivered back to the users, optionally integrated with antibiotic sensitivity and virulence results if extended to multi-task classification.

In one aspect, the invention provides a system for pathogen identification (preferably in a cloud environment), the system comprising:
- a memory storing instructions for performing a Fourier Neural Operator-based classification pipeline on MALDI spectral data;
- a processor configured to:
   (a) receive digital spectra (in some embodiment, from remote laboratories),
   (b) preprocess the spectra,
   (c) execute at least one Fourier layer that applies a forward Fourier transform to obtain transformed feature representation, multiply the transformed feature representation by learned complex multipliers to obtain convoluted feature representation, and apply an inverse Fourier transform to the convoluted feature representation to obtain feature vectors;,
   (d) output species probabilities;
- a communication interface enabling (optionally remote) laboratories to request classification and retrieve pathogen results.

The instructions according to the method of the invention preferably cause the processor to integrate a baseline correction, a smoothing filter, and uniform binning steps prior to Fourier layer operations.

The system may further comprise an antibiotic resistance and virulence prediction module configured to use a multi-task network head, thereby producing multiple labels in a single inference pass.

In an aspect, the invention provides a non-transitory computer-readable medium storing instructions that, when executed by at least one processor, cause the steps of the method of the invention to be carried out.

Furthermore, the invention provides a method for updating the system, comprising the following steps:
- collecting newly labeled MALDI spectra for one or more pathogen species,
- augmenting the training dataset with said new samples,
- re-training or fine-tuning the spectral convolution layers.

Preferably, the re-trained model is validated on an internal hold-out set to confirm improvements in classification accuracy before being made publicly available to all authorized laboratory clients.

### DETAILED DESCRIPTION OF THE INVENTION

The method begins when a laboratory obtains a MALDI-TOF spectrum of a clinical or environmental sample. The cloud-based system receives digital spectra data, typically representing intensity values mapped to m/z values. The system processes these intensities into bins, and processes the bins through an FNO-based pipeline designed to learn mappings from these spectral function spaces to discrete pathogen classes.

Each MALDI spectrum s(x) can be viewed as a function: $s : \left[m/zmin,m/zmax\right] \to R$ indicating the intensity at each m/z. This domain is preferably discretized into N bins. Traditional CNNs handle these bins as positions along a "1D image," but the FNO treats them as points in a function space.

The pre-processing procedure may contain one or more of the following steps:
(a) Baseline Correction: A morphological or rolling average technique subtracts the baseline drift.
(b) Smoothing: A Savitzky-Golay or similar filter reduces high-frequency noise, improving signal clarity.
(c) Normalization: The system can apply total ion current (TIC) normalization, scaling intensities so that the integrated intensity sums to one or a chosen constant.
(d) Bin Definitions: Typically, the range from e.g. 2,000 to 20,000 Da is divided into bins of size 50 Da each. The intensities in each bin are summed or averaged, producing a vector v ∈ R^{N}.

The Fourier Neural Operator and neural network processing can in some embodiments be described as follows:
(a) Lifting Layer: The vector v is expanded from shape (N) to (width,N) through a linear transform. This "width" dimension acts as feature channels for subsequent spectral operations.
(b) Spectral Convolution: Each spectral layer performs the following steps:
   - FFT along the N dimension, generating a representation *V̂* in the frequency domain.
   - Truncation or focus on the first modes frequency components to limit computational overhead.
   - Learned complex multipliers for each frequency mode, essentially weighting amplitude and phase for each channel.
   - Inverse FFT to revert to the "spatial" domain (the bin axis), now refined by global frequency interplay.
(c) Residual/Activation: The output of the spectral convolution is combined with local linear transforms (like 1×1 convolutions) in the binned domain, plus a ReLU or similar activation function. Dropout or batch normalization can be integrated to mitigate overfitting.
(d) Global Pooling: After one or more spectral layers, the final feature map is average-pooled across N bins, resulting in a width-dimensional vector that captures global spectral content.

The classification layer may have the following architecture:
The pooled feature vector enters a fully connected layer (or layers) that produce logits for each pathogen class. For multi-class classification with C potential pathogens, the final dimension is C. A softmax transforms these logits into probabilities: $P \left(species=i\right) = \frac{exp \left({logit}_{i}\right)}{{\sum }_{j = 1}^{C} exp \left({logit}_{j}\right)}$

The predicted species is the argmax of these probabilities.

Alternative or additional embodiments may include:
(a) Multi-Task Classification:
   The same pipeline can produce antibiotic resistance and virulence predictions if antibiotic and virulence labels are available. Another output head can produce, for example, a multi-label classification for resistant (R), sensitive (S) or intermediate (I) statuses or virulence statuses.
(b) Variable Binning:
   The bins can be formed based on fixed or variable bin size (fixed m/z interval size), or an adaptive or learned binning approach might be employed. The FNO concept still applies, as we ultimately treat the final shape as (width,N).
(c) Iterative Refinement:
   Some embodiments may apply additional loops of the spectral layers, effectively deepening the operator's coverage. The invention contemplates such variants as consistent with the global coverage principle.

Typical metrics used to gauge the system's effectiveness include:
- Accuracy or top-k accuracy across a validated set of species.
- Precision, recall, F1 scores for each species, especially relevant in multi-class contexts.
- Inference latency on the cloud platform, typically a few hundred milliseconds to a few seconds depending on concurrency levels.

In a preferred embodiment, adaptive cloud-based inference may have the following features:
(a) Deployment:
   The model weights for the FNO are deployed on a secure cloud server. Laboratories can upload binned (or raw) MALDI data through a web portal or an API call. The server runs the FNO pipeline, returning classification results to authorized users.
(b) Scalability:
   Using horizontally scalable containerization or orchestration frameworks, additional compute instances can be spun up to handle surges in sample volume. FNO computations, while heavier than some local methods, can be distributed across multiple GPUs in the cloud environment.
(c) Continuous Learning:
   Potentially, new pathogen reference spectra can be integrated, retraining or fine-tuning the FNO model, with the updated model seamlessly redeployed in the cloud so all users benefit from improved coverage.

The present invention provides a system and method for identifying microbial pathogens from MALDI-TOF mass spectrometry data using a Fourier Neural Operator (FNO) approach deployed on a cloud-based platform. The invention treats each MALDI spectrum as a global function, performing baseline correction, smoothing, and binning. A neural operator architecture applies learned transformations in the frequency domain, capturing wide-range spectral dependencies. The final global feature vector is classified into pathogen species. This yields more robust performance than local convolutional filters, especially for subtle distinctions among closely related microorganisms. The cloud-based digital platform supports high-throughput sample uploads and real-time inference, enabling a scalable, centralized service that streamlines clinical or research workflows in microbiological diagnostics.

### EXAMPLE OF CARRYING OUT THE INVENTION

Detailed Steps of the Method in one example
Data Acquisition:
   ▪ MALDI-TOF device obtains raw spectra. Lab staff uploads data to a cloud-based platform.
Preprocessing:
   ▪ The system performs baseline correction with a morphological opening method.
   ▪ Then a smoothing filter (like Savitzky-Golay) is applied.
   ▪ Next, intensities are normalized by the total area under the curve.
Binning:
   ▪ The domain from 2,000 Da to 20,000 Da might be split into 360 bins of 50 Da each, producing a 360-length vector of intensities.
Lifting:
   ▪ A linear transform expands from shape (360) to (width=128, 360).
Fourier Layers:
   ▪ Each spectral layer computes an FFT along dimension 360, keeps e.g. 64 modes, multiplies complex weights, then iFFT.
Pooling and Classification:
   ▪ After e.g. four repeated spectral layers, a global average pooling yields (128).
   ▪ A fully connected layer of size (128 × 50) might produce 50 pathogen logits, turning into probabilities.
Result:
   ▪ The species with maximum probability is selected as the predicted pathogen. The cloud platform returns the classification result via a user dashboard.

### System Overview

The system is divided into two primary pipelines:
Training Pipeline: Ingests and validates spectra submitted by users, adds validated data to a secure training dataset, retrains the ML model periodically, and manages versioned model artifacts.
Classification Pipeline: Provides real-time classification through an API, ensuring authentication, licensing checks, and scalable model serving.

A preferred cloud deployment may be provided, or an offline deployment may be provided to allow users to run the model locally in a secure, containerized environment with encrypted model parameters.

### Architecture Components- AWS Infrastructure overview

- The core of the system resides in a VPC (virtual private cloud) where all the training, evaluation and data storage reside. The VPC only allows a small subset of requests coming from the rest of the application and no general permissions.
- The main secure storage for the system is an S3 bucket for storing large amounts of training data, and a history of trained and deployed models.
   ∘ Staging bucket resides outside the VPC and its purpose is to ingest user data
   ∘ Data bucket resides inside of the VPC and is the main storage area for the system
- The VPC also hosts a Postgres instance RDS which serves as the main database. The purpose of the main database is
   ∘ Indexing all training data, training and validation sets, pointing to the raw files on S3
   ∘ Copy of the user database (main user database is AWS cognito) with licencing information
- Serverless control cluster is distributed both within the VPC but also outside where it can perform. The control cluster is defined by a network of cooperating Lambda functions which:
   ∘ Verify the uploaded data and construct training and validation sets
   ∘ Verify user Access Token and map them on licencing details of the user account
   ∘ Control the training and deployment cluster
- Worker cluster resides also within the VPC. The worker cluster consists of a number of EC2 nodes whose purpose is to perform the training of the models and evaluation of the user requests.
- Apache Kafka is placed between the Worker nodes and the Control cluster to manage and throttle the requests
- API Gateway with load balancer is put in place to send and communication and data from the user and it distributes the traffic among nodes in Control Cluster and Worker Cluster
- Identity management is done via AWS Cognito which maintains a pool of registered users. User access to the data and or services is managed via JWT access tokens.
- User application that runs on their hardware and interacts with our system

In one preferred embodiment, each user site uses a standardized local script to convert raw MALDI data into a bin vector of length 400. The site uploads this vector, along with minimal metadata, to the cloud. The FNO model in the cloud uses a width of 128 channels and up to 64 frequency modes. Once classification is computed, the result is automatically returned, typically within a few seconds. This approach ensures minimal local infrastructure demands while maximizing the efficiency of the central operator-based classification system.

### Workflows and pipelines

### User data submission pipeline

Assuming the user has a valid account and is authenticated with AWS Cognito
- User launches the application on the MALDI-TOF device
- User logs in with their credentials. User login info is sent securely via HTTPS
- User selects one or more files to be uploaded to the system.
- User application sends a file upload request to the API gateway
- Control cluster node that is available picks the request up and validates the user token and generates a pre-signed S3 upload request to the staging bucket and sends it to the user. In the case that the user authentication fails, the user receives an error
- User application receives the pre-signed upload request and uses it to upload selected MALDI-TOF spectra with their classifications to the Staging Bucket. Upload is logged
- Once uploaded the user receives the confirmation of the upload and the upload is logged into the SQL database
- Free control cluster node is triggered by the upload and performs data validation and moves the data into Data Bucked and logs this in the main SQL database. The Organization of the user who uploaded the data is given one upload credit which is logged in the SQL database. Confirmation message is sent towards the user that the data have been validated.
For the system operator, the ingestion data can also be done with a direct upload to S3 or via the Operator Application which is a web based application hosted on AWS amplify. The administrators are normal users and reside in the same database but have elevated privileges.

### Training pipeline

- Once enough new data has been collected a free CC node launches rendering of the training set from the S3 into the ML format supported by PyTorch. The final version of the training sets is distributed to the Worker nodes that will do the training.
- The CC Node schedules the resources for the training and sends a request to the WC nodes to commence the training. Worker nodes commence training of the model while periodically reporting to the Control Cluster.
- The Admin user can oversee the training process
- Once training is done a validation is performed also by the Worker nodes.
- After validation the model is versioned and its parameter se ti stored in the Data Bucket and logged in the SQL database

### Component details

### Data Storage

- S3 Buckets:
   Two buckets are used for different stages:
   ∘ Staging Bucket
      All spectra submissions are initially loaded here from client apps (desktop and web).
   ∘ Data Bucket
      Once validated by Lambda functions, spectra are moved to this secure bucket. It also stores versioned, trained model definitions.
- SQL Database (RDS - PostgreSQL):
   Stores indexing information for the training set and model versions. Running within the VPC, it provides a secure, managed relational database solution.

### Compute and Machine Learning Resources

- Worker - Training - Cluster:
   ∘ EC2 Instances (GPU-enabled):
      A suitable instance type for a 10-100-million-parameter model might be the p3.8xlarge or a_p4d instance, which offer powerful GPUs and ample memory.
   ∘ PyTorch Environment:
      The training process leverages PyTorch on these instances.
   ∘ VPC Placement:
      EC2 instances reside in a private subnet for added security.
- Worker - Runner - Cluster:
   ∘ EC2 Instances (Production Serving):
      These are less resource-intensive instances compared to the training cluster but are scaled behind a load balancer.
   ∘ Load Balancer and API Gateway:
      ▪ API Gateway:
         Acts as the entry point for classification requests.
      ▪ Load Balancer:
         Distributes requests to the runner EC2 instances. Prior to request dispatch, the JWT access token is verified and a licensing Lambda checks available classification quotas.

### Advantages Over Local Receptive Field Methods

1. Global Patterns:
   The entire range of m/z bins is considered simultaneously via the Fourier transform, enabling detection of subtle global relationships, such as correlated peaks at distant bin positions.
2. Reduced Overfitting:
   Because the FNO approach distributes learning across frequency modes, it can better handle shifts or noise that break purely local structures. This is especially relevant for MALDI data, where small variations can arise from sample prep or device calibration.
3. Scalable Cloud Service:
   Users do not require on-premise GPU clusters. The invention simplifies integration by hosting the model in the cloud, automatically distributing workload as needed.

### Preferred features and embodiments:

- Real-Time Monitoring: Once integrated into a laboratory's pipeline, the invention can provide near real-time alerts for critical pathogens.
- Adaptive Bin Sizing: Instead of uniform bins, the system could incorporate variable bin sizes to capture peak-dense regions more finely.
- Transfer Learning: If new species data becomes available, partial re-training or fine-tuning of the spectral layers may be performed, updating the cloud model and instantly benefiting all connected labs.

### Practical Implementation Example

- For example, a hospital microbiology lab obtains 100 MALDI spectra per day from suspected bacterial isolates.
- Each spectrum is automatically preprocessed by local scripts, then transmitted to the cloud FNO classification endpoint.
- The system returns a top-5 list of probable species with probabilities, typically within seconds or minutes, thereby aiding the microbiologist in selecting confirmatory tests or deciding on immediate antibiotic therapy.

### Example of System Architecture

- User/Client: Desktop application or user interface.
- Cloud/Server Layer:
   1. Preprocessing Service: Confirms data format, handles minimal transformations if necessary.
   2. FNO Inference Service: Contains loaded FNO model with weight parameters.
   3. Database: Stores metadata about each run.
- Output: A classification response with predicted pathogen, confidence scores, or recommended next steps.

## Claims

1. Method of identifying a microbial pathogen according to the invention comprised the following steps:
- obtaining a MALDI mass spectrometry spectrum representing intensities at m/z;
- preprocessing said spectrum by at least one of baseline correcting, smoothing, and normalizing intensities;
- performing a lifting transform to map said one-dimensional intensities into a higher-dimensional feature representation (space);
- executing at least one Fourier layer that applies a forward Fourier transform to obtain transformed feature representation, multiplying the transformed feature representation by learned complex multipliers to obtain convoluted feature representation, and applying an inverse Fourier transform to the convoluted feature representation to obtain feature vectors;
- pooling feature vectors across bins to produce a global representation;
- classifying the global representation into a pathogen label.

2. Method according to claim 1, which includes obtaining a MALDI mass spectrometry spectrum representing intensities at m/z and processing the MALDI spectrum as follows:
a) a preprocessing step including MALDI spectra wherein the preprocessing step comprises at least one of baseline correction, smoothing and normalization;
b) optionally a step of dividing the MALDI spectral data into intervals (bins) of m/z values and calculating an average intensity in each interval (bin);
c) processing of the pre-processed MALDI data by a Fourier Neural Operator Architecture which comprises several Fourier layers, wherein c1) a lifting layer maps the one-dimensional MALDI data into a higher-dimensional feature space and wherein the c2) Fourier layers transform the obtained features in the higher-dimensional feature space to a frequency domain,
d) processing the obtained features in the higher-dimensional feature space by a neural network applying learned complex multipliers,
e) processing the data obtained in step d) by inverse Fourier transformation to obtain feature vectors,
f) repeating the steps c2) to e) at least twice, preferably at least four times, wherein the input of the repeated step c2) are the data obtained in step e);
g) pooling feature vectors across bins to produce a global representation;
h) classifying the global representation into a pathogen label.

3. A system for pathogen identification, the system comprising:
- a memory storing instructions for performing a Fourier Neural Operator-based classification pipeline on MALDI spectral data;
- a processor configured to:
(a) receive digital spectra,
(b) preprocess the spectra,
(c) execute at least one Fourier layer that applies a forward Fourier transform to obtain transformed feature representation, multiply the transformed feature representation by learned complex multipliers to obtain convoluted feature representation, and apply an inverse Fourier transform to the convoluted feature representation to obtain feature vectors;,
(d) output species probabilities;
- a communication interface enabling users to request classification and retrieve pathogen results.

4. The system of claim 3, wherein the instructions cause the processor to integrate a baseline correction, a smoothing filter, and uniform binning steps prior to Fourier layer operations.

5. The system of claim 3 or 4, further comprising an antibiotic resistance and virulence prediction module configured to use a multi-task network head, thereby producing multiple labels in a single inference pass.

6. A non-transitory computer-readable medium storing instructions that, when executed by at least one processor, cause the steps of any of claims 1 to 2 to be carried out.

7. Method for updating the system of any one of claims 3 to 5, comprising the following steps:
- collecting newly labeled MALDI spectra for one or more pathogen species,
- augmenting the training dataset with said new samples,
- re-training or fine-tuning the spectral convolution layers.

8. The method of claim 7, wherein the re-trained model is validated on an internal hold-out set to confirm improvements in classification accuracy before being made publicly available to all authorized laboratory clients.
